Europäisches Patentamt

European Patent Office

Office européen des brevets

(11) Publication number: **0 550 683 B1**

## EUROPEAN PATENT SPECIFICATION

(45) Date of publication of patent specification: **19.04.95**   (51) Int. Cl.⁶: **C07K 14/00**, A61K 39/10

(21) Application number: **91919318.5**

(22) Date of filing: **25.09.91**

(86) International application number:
**PCT/NL91/00185**

(87) International publication number:
**WO 92/05194 (02.04.92 92/08)**

(54) **VACCINE SUITABLE FOR COMBATTING BORDETELLA PERTUSSIS.**

(30) Priority: **25.09.90 NL 9002092**

(43) Date of publication of application:
**14.07.93 Bulletin 93/28**

(45) Publication of the grant of the patent:
**19.04.95 Bulletin 95/16**

(84) Designated Contracting States:
**AT BE CH DE DK ES FR GB GR IT LI LU NL SE**

(56) References cited:
**WO-A-90/12086**
**WO-A-91/15505**

**BIOLOGICAL ABSTRACTS, vol. 88, 1989; M.G. THOMAS et al., no. 4699&NUM;**

**JOURNAL OF BACTERIOLOGY, vol. 159, no. 2, August 1984, American Society for Microbiology; F. SHARECK et al., pp. 780-782&NUM;**

(73) Proprietor: **DE STAAT DER NEDERLANDEN VERTEGENWOORDIGD DOOR DE MINISTER VAN WELZIJN, VOLKSGEZONDHEID EN CULTUUR**
**P.O. Box 5406**
**NL-2280 HK Rijswijk (NL)**

(72) Inventor: **HAMSTRA, Hendrik-Jan**
**Rosmolen 36**
**NL-3961 LJ Wijk Bij Duurstede (NL)**
Inventor: **POOLMAN, Jan, Teunis**
**Leeteinde 8**
**NL-1151 AK Broek in Waterland (NL)**

(74) Representative: **de Bruijn, Leendert C.**
**Nederlandsch Octrooibureau**
**Scheveningseweg 82**
**P.O. Box 29720**
**NL-2502 LS Den Haag (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

JOURNAL OF BACTERIOLOGY, vol. 166, no. 1, April 1986, American Society for Microbiology; S.K. ARMSTRONG et al., pp. 212-216&NUM;

REVIEWS OF INFECTIOUS DISEASES, vol. 10, suppl. 2, July-August 1988, University of Chicago; C.D. PARKER et al., pp. S327-S330&NUM;

PROCEEDINGS OF THE NATL. ACADEMY OF SCIENCES USA, vol. 86, no. 10, May 1989, Washington, DC (US); I.G. CHARLES et al., pp. 3554-3558&NUM;

## Description

The invention primarily relates to a vaccine which is suitable for combating B. pertussis in humans.

Prior art

As reported in The Lancet, June 2, 1990, pp. 1326-1329, Bordetella pertussis is the causative organism of whooping cough. More particularly, B.pertussis is a Gram-negative bacterium which adheres to the cilia of epithelial cells in the human respiratory tract, where the bacterium reproduces and gives off toxic substances. At the local level, the infection causes destruction of the epithelium of the respiratory tract and kills the cells provided with cilia, which gives rise to, for example, impeded breathing, paroxysmal coughing, apnoea and encephalopathy, which may or may not be accompanied by fever. The infection can occur in humans at any age but occurs mainly in infants and young children.

Although B.pertussis is sensitive to a variety of antibiotics, treatment with such antibiotics is not effective since, before the disease is diagnosed, the B.pertussis bacteria have often already attacked the respiratory tract and have given off the toxins which are responsible for the serious consequences brought about by the disease. Therefore, preventive protection against B.pertussis is a matter of crucial importance.

In the light of the above, vaccines have been developed, the "whole-cell" vaccine of B.pertussis having been used as first vaccine. A vaccine of this type consists of whole B.pertussis cells, which have been killed, for example by the action of heat or a formalin treatment. This type of vaccine is effective in preventing whooping cough. Nevertheless, the use of this type of vaccine is highly controversial because of the occurrence of local and systemic side effects. Some effects are mild reactions, which also occur with other vaccines, but nevertheless occur more frequently in the case of B.pertussis vaccines. Examples of such effects are redness, pain, induration and fever. The B.pertussis "whole-cell" vaccine also causes other reactions specific for this type of vaccine, such as persistent crying and convulsions. Moreover, severe side effects such as brain damage and even death also arise with this type of vaccine.

In the light of the above-described disadvantages associated with B.pertussis "whole-cell" vaccines, which vaccines contain a variety of substances such as, for example, proteins, nucleic acids, peptidoglucans, lipids and lipopolysaccharides, a broad-based study has been started to develop acellular vaccines, with which, in the optimum case, only those antigens which generate adequate immunity and themselves display no toxicity should be allowed to be present.

The generally recognised intracerebral (i.c.) mouse protection test [Medical Research Council. 1956, Vaccination against whooping cough. Relation between protection tests in children and results of laboratory tests. Brit.Med.J. 2: pp. 454-462] is chosen as a criterion for the effectiveness of such acellular vaccines.

As can be seen from the abovementioned article in The Lancet, vaccines containing various antigens have been tested. One of the examples is detoxified pertussis toxin (PT toxoid) which apparently - according to the i.c. mouse protection test - is less effective than the "whole-cell" vaccine. Only when one or more other antigens, such as, for example, the outer membrane protein (OMP) having a molecular weight of 69kDa (69K OMP) are added is a potency achieved which to some extent approaches that of the "whole-cell" vaccine. One example of such acellular vaccines is, inter alia, a mixture of filamentous haemagglutinin (FHA) and pertussis toxin (PT), which have been obtained together from culture supernatants and detoxified with the aid of formaldehyde. However, there is always the risk with the use of such detoxified PT-containing vaccines that a reversion to toxicity takes place during the clinical treatment. For this reason, genetically deactivated forms of pertussis toxin have been developed. In this case, the B.pertussis toxin gene has been modified by means of deletion, insertion or substitution of codons coding for specific amino acids in such a way that the PT then has hardly any toxicity but has retained the immunity-generating action [Pizza, M.G. et al., Mutants of pertussis toxin suitable for vaccine development. Science 246, (1989), pp. 497-500].

INVENTION

In view of the problems described above, the Applicant has carried out a study to find effective, non-toxic acellular B.pertussis vaccines which - after administration - provide an effective protection against B.pertussis in humans.

Vaccines suitable for combating B.pertussis have been found, which vaccines comprise, as active components, at least one outer membrane protein (OMP) having a molecular weight of about 92kDa or 32kDa derived from B.pertussis, which OMP or OMPs are present in an outer membrane vesicle (OMV) formulation or an artificial vesicle formulation. Examples of artificial vesicle formulations are, for example, a liposomal, proteosomal and detergent micelles.

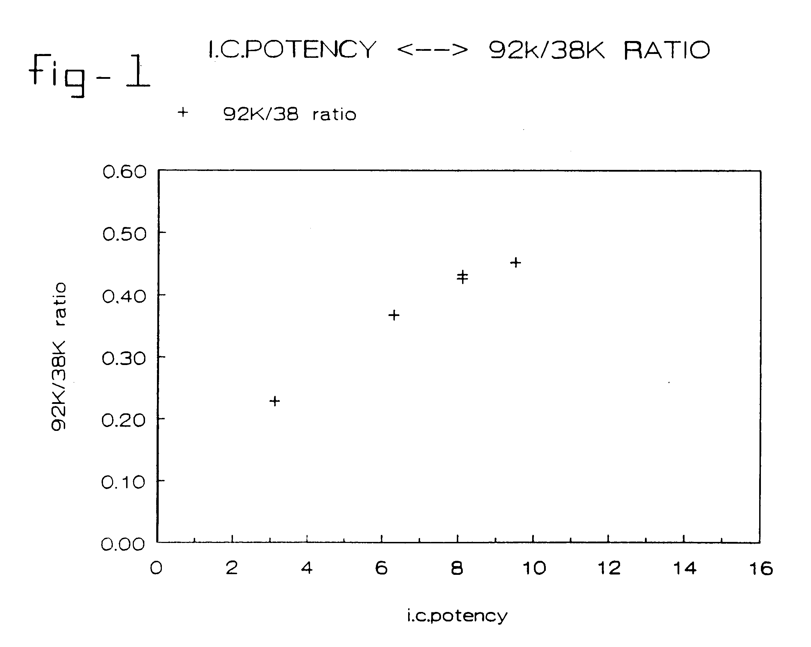

In the i.c. mouse protection test, vaccines of above type give values which are well above the internationally specified minimum protection value of 4. Further studies carried out by the Applicant have shown, surprisingly, that a relationship exists between the ratio between the amounts of OMPs having a molecular weight of 92kDa and 32kDa (92K OMP and 32K OMP) and the OMP having a molecular weight of 38kDa (38K OMP). on the one hand, and the efficacy or protection value (according to the i.c. mouse protection test), on the other hand. On the basis of these findings, the 92K OMP/38K OMP weight ratio must, according to the Applicant, be at least greater than 0.25 and advantageously greater than or equal to 0.4. for example 0.5 or even higher. The relationship concerned is shown as a graph in Fig. 1. OMPs as well as OMVs having an optimum OMP-ratio can be obtained by means of genetic engineering. OMPs as well as OMVs having optimum ratios of 92kDa and 32kDa OMPs relative to the 38kDa OMP, which is indispensable for growth, can be prepared by this generally known technique.

Furthermore, the invention advantageously relates to artificial vesicles in which, preferably, at least the 32kDa OMP and/or the 92 kDa OMP is present. An OMP of this type can be brought into the form of so-called "mixed" protein-detergent micelles with the aid of a detergent such as Zwittergent® 3-14. and also other detergents like Triton® X-100 and octylglucoside. According to the i.c. mouse protection test. artificial vesicles of this type have valuable activities. as is indicated below in Table 3.

Also other ingredients which are suitable as vaccine ingredients can be added to the vaccines in question. Examples of such ingredients are for example adjuvants like AlPO$_4$.

With a view to the presence of lipopolysaccharides in OMVs, which (can be) are active as endotoxins, the invention also relates to vaccines which comprise OMVs which have been treated with an effective amount of desoxycholate in order to lower the endotoxin content.

Better definable vaccines according to the invention can be obtained with the aid of fimbriae⁻ and/or FHA⁻ B.pertussis strains. Such strains can be selected in a simple and generally known manner.

The invention also relates to vaccines whose efficacy has been increased by the addition of additional active components such as PT toxoid and/or FHA. Although the PT toxoid can be derived from PT detoxified using formaldehyde or glutaraldehyde, the PT toxoid preferably used is that which has been extracted from B.pertussis strains in which the PT gene has been modified in such a way that it yields a non-toxic PT product of adequate immunological activity.

The vaccines according to the invention are used for the vaccination of humans, in particular children less than 2 years old. The dose to be injected can be about 5-25 $\mu$g OMV.

Finally, the invention relates to the above-defined OMPs and OMVs, derived from B.pertussis strains, which can be used for the preparation of the vaccines according to the invention.

## Detailed description of the invention

Many B.pertussis antigens, such as detoxified PT, filamentous haemagglutinin (FHA) fimbriae, virulent stage OMPs and avirulent stage OMPs were reviewed in the study carried out by the Applicant. All of these antigens were tested using the intracerebral (i.c.) mouse protection test to determine their activity. This is because it has been found that this internationally known i.c. mouse protection test is correlated to the field protection.

The abovementioned antigens, which differ from PT toxoid, were obtained from a PT-negative mutant of B.pertussis, specifically the BP-TOX6 mutant, as described in Relman, D.A. et al., Filamentous hemagglutinin of Bordetella pertussis: nucleotide sequence and crucial role in adherence. Proc.Natl.Acad.Sci 86 - (1989), pp. 2637-2641. This BP-TOX6 mutant lacks the pertussis toxin operon. More particularly, the following antigens were obtained from this mutant: purified FHA, fimbriae, OMVs and 92K OMP, 69K OMP, 38K OMP and 32K OMP.

The results of the B.pertussis antigens tested and of a "whole-cell" B.pertussis vaccine (WCV) are given in Table 1 below.

4

TABLE 1

| | |
|---|---|
| KH 85/1 (whole-cell vaccine) | 6.0 |
| Pertussis toxoid (formaldehyde) | 12.8 |
| Pertussis toxoid (9K/129 G mutant) | 14.9 |
| FHA (BP-TOX6) | 11.8 |
| OMV (BP-TOX6) | 6.3 |
| OMV/DOC (BP-TOX6) | 4.3 |
| OMV (BP-TOX6, C mode) | 1.3 |
| OMV (BP 509) | 8.1 |
| OMV (BP 509, C mode) | 2.7 |
| OMV (BP 509, fim⁻ mutant) | 9.8 |
| OMV (BP 509, FHA⁻ mutant) | 6.2 |
| OMV (BP 134) | 9.5 |
| OMV (BP 134, C mode) | 3.1 |
| OMV (BP W28) | 3.1 |
| OMV (BP W28, 69K⁻ mutant) | 5.0 |
| fimbriae 2 + 3 | ND |
| 69K OMP | ND |
| 92K OMP | ND |
| 38K OMP | ND |
| 32K OMP | ND |

ND = not detectable C mode: avirulent stage fim⁻ mutant: mutant, which has no fimbriae FHA⁻ mutant: mutant which has no FHA DOC: subjected to desoxycholate treatment

As can be seen from Table 1 above, a number of antigens have a protection value of ≥ 4. These include PT toxoid (either treated with formaldehyde or the mutated non-toxic PT), FHA and OMV. The purified fimbriae and OMPs having a molecular weight of 92kDa (92K OMP), 69kDa (69K OMP), 38kDa (38K OMP) and 32kDa (32K OMP), however, had no measurable protection value.

Tests on various preparations, analysed using SDS-PAGE and electroblotting after colouring with monoclonal antibodies (anti-PT, anti-FHA, anti-fimbriae, anti-92K OMP, anti-69K OMP, anti-38K OMP and anti-32K OMP) for the specific proteins or Coomassie Blue® for the total protein, showed that
- the WCV preparation essentially contains outer membrane proteins,
- OMV in the avirulent stage contains 38K OMP, 33K OMP and 18K OMP, and
- OMV in the virulent stage contains 92K OMP, 32K OMP and 30K OMP.

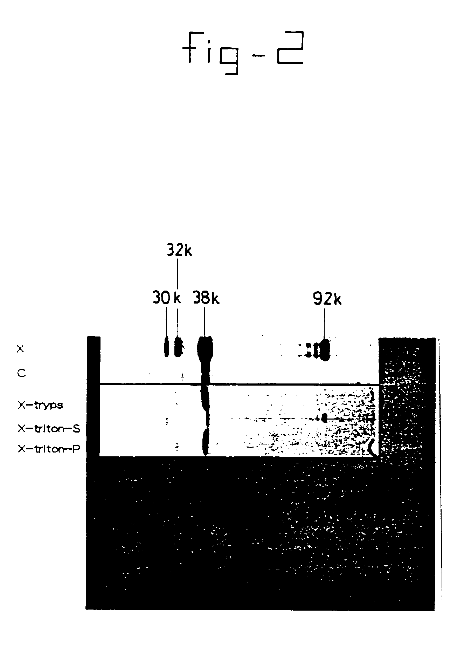

It can be seen from the appended Fig. 2 that the avirulent stage OMPs (38, 33 and 18kDa) can be differentiated from the virulent stage OMPs (92, 32 and 30kDa) on the basis of trypsin sensitivity and Triton® X-100/MgCl$_2$ extractability.

The following is pointed up with regard to the endotoxin content present in the preparations tested. The endotoxin content in the FHA preparation was considered high by the Applicant. The WCV and OMV vaccines have comparable endotoxin contents. It proved possible to lower this endotoxin content of the OMV preparations by a factor of $10^4$ using the desoxycholate (DOC) treatment described in more detail below. This DOC treatment gave rise to a reduction in vesicle size of 40-180 nm (OMV) to 10-25 nm (OMV-DOC).

The results of serological analysis of antibodies generated in mice, using a single dose immunisation with WCV, OMV, PT$_x$, FHA, fimbriae, 92K OMP, 38K OMP and 32K OMP, are shown in Table 2 below.

TABLE 2

| Antibody response in mice immunised with WCV, OMV, fimbriae, FHA and $PT_x$ ELISA antigen | | | | | | | |
|---|---|---|---|---|---|---|---|
| Vaccine | WC* | OMV | LPS | fim 2 | fim 3 | PT | FHA |
| WCV 12.5 | 1.721 | 2.221 | 0.093 | 0.085 | 0.038 | 0.030 | 0.046 |
| $PT_x$(form) | 0.750 | 0.871 | 0.059 | 0.110 | 0.097 | 2.310 | 0.479 |
| $PT_x$(mut.) | 0.305 | 0.282 | 0.048 | 0.080 | 0.072 | 2.996 | 0.285 |
| FHA | 0.498 | 1.031 | 0.026 | 0.018 | 0.066 | 0.043 | 2.443 |
| OMV | 2.616 | 3.270 | 0.648 | 0.633 | 0.185 | 0.038 | 0.699 |
| fim 2 + 3 | 2.508 | 3.169 | 0.037 | 1.826 | 1.075 | 0.217 | 0.063 |
| 92K | 2.035 | 1.986 | 0.056 | 0.188 | 0.102 | 0.178 | 0.069 |
| 38K | 1.484 | 2.399 | 0.024 | 0.146 | 0.057 | 0.095 | 0.117 |
| * "Whole-cell" preparation for ELISA testing was prepared from shaking-bottle cultures. | | | | | | | |

It can be deduced from the above Table 2 that the prepared WCV vaccine (in the virulent stage) yields no detectable antibody formation against PT, FHA and fimbriae. $PT_x$, FHA and fimbriae induce antibodies, as detected by WC and OMV. The WC and OMV preparations, used as coating antigen, contain some FHA and fimbriae, while FHA and fimbriae preparations contain certain OMP.

OMV vaccines induce some antibodies against fimbriae and FHA. In order to prevent this effect, the Applicant used fimbriae⁻ and FHA⁻ variants of the 509 strain, obtained by means of colony blotting using monoclonal antibodies, in its study. Variants of this type are illustrated in Fig. 3. OMV vaccines, prepared from such variants, had protection values similar to those of OMV vaccines prepared from the wild type 509 strain.

OMV vaccines prepared from other tested B.pertussis strains in the virulent stage, such as the PT⁻ strain BP-TOX6, strain 134 and the 69kDa⁻ mutant of the W28 strain, have an adequate i.c. mouse protection value. When the B.pertussis strains BP-TOX6, 509 and 134 were in the avirulent stage, the i.c. mouse protection value of the OMV vaccines decreased sharply. In this context it is emphasised that the difference between the OMVs in the virulent and the avirulent stage lies in the presence, discussed above, of the 92K OMP, 32K OMP and 30K OMP in the virulent stage. The greater the 92K OMP and 32K OMP content relative to the 38K OMP content, the greater is the i.c. mouse protection value of the OMV vaccines (see Table 1 and Fig. 1). The preference for a high 92K OMP/38K OMP ratio in OMV vaccines is clearly apparent from Fig. 1, which shows the critical value of 4 with regard to the protection potency. Above aspect may also be expressed in the 32K OMP/38K OMP ratio as the 92K OMP/32K OMP ratio is constant in non-manipulated B.pertussis strains (see Figure 3). In Table 3 below, the results of experiments i.e. the i.c. mouse protection test are reported which are performed with purified 92K OMP, 38K OMP and 32K OMP in a Zwittergent®-3,14 solution in the presence and absence respectively of pertussis toxin (PT). From the results of these experiments with these artificial vesicles it can be deduced that the purified 32K OMP and 92K OMP in a correct formulation provide a sufficient activity against a B.pertussis challenge.

TABLE 3

| i.c. potency of purified OMPs | | |
|---|---|---|
| Antigen | PT | Surviving mice (10) |
| 92K + Zw* 3-14 | - | 2/2/2/4 |
| 92K + Zw 3-14 | + | 8/8/5/1 |
| 32K + Zw 3-14 | - | 8/5/1/2 |
| 32K + Zw 3-14 | + | 10/8/7/4 |
| 38K + Zw 3-14 | - | 1/3/4/6 |
| 38K + Zw 3-14 | + | 4/8/3/1 |
| * Zw = Zwittergent | | |

In the above table, column 1 shows the antigen used, column 2 the presence ( + ) of PT or absence (-) of PT and column 3 the survival rate of the mice (10 per test).

The amino acid sequences of a few OMPs, which were determined with the aid of a gas phase sequencer, are given below.

```
(a)  92kDa OMP
     AAVTAAQRIDGGAAFLGDVAIAT(T)K(A)(S)(E)
                                (A)

(b)  32kDa OMP
     ALSKRMGELRLTPVAGGV(W)(G)(R)AF(G)
                               (V)

(c)  33kDa OMP
     ALSKRMGELRLTPVAGGVWGRAFV (G)(Y)(Q)
                              (G)(R)(R)(V)
                              (N)    (L)

(d)  30kDa OMP
     ALDKRLGELRL(N)A DAG (G)---
                         (P)

(e)  38kDa OMP
     (E)TSVTLYGIIDTGI(G)YNDV(D)FKV(K)GANA-(D)
                                (T)
```

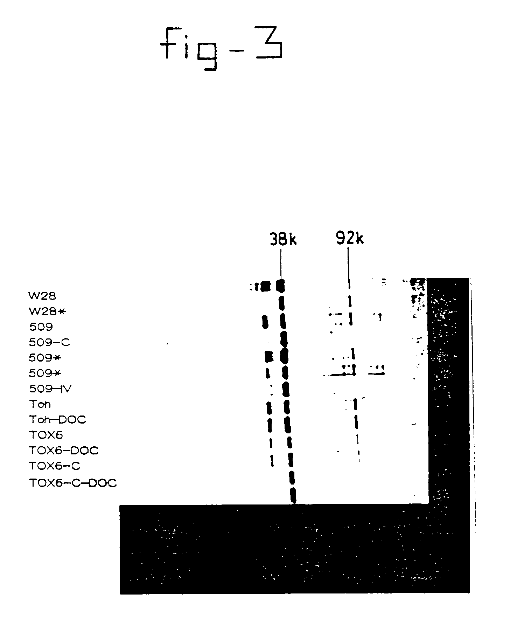

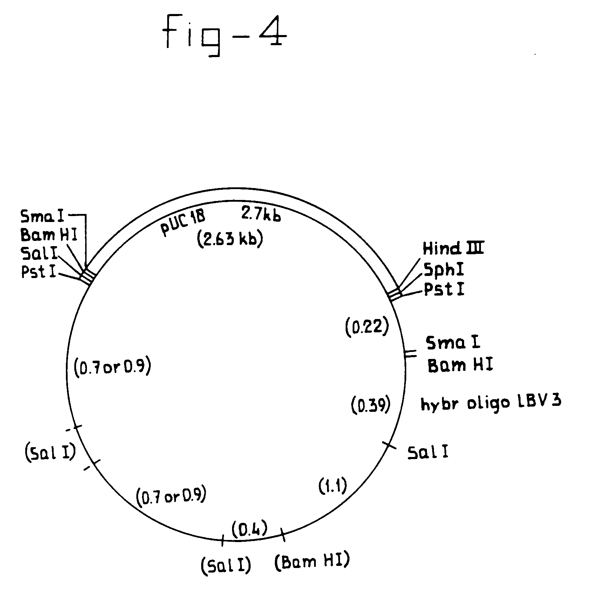

In view of the above it is considered desirable to produce the 92 kDa and 32 kDa OMPs preferably by B.pertussis or optionally other microorganisms. This production can be realized by cloning the relevant structural genes and expressing these genes at an increased level; this last-mentioned production method can be considered standard technology. An example of the above standard technology is elucidated in Figure 4. More in particular Fig. 4 illustrates the well-known plasmid pUC18 having a 3.8 kb insert comprising the 32 kDa OMP gene. This structural gene is obtainable from the gene bank (Mooi et al, 1987, Microbial. Pathogenesis, 2, pp. 473-484) with the help of a nucleotide sequence derived from the N-terminus of the 32 kDa OMP:

5' ACCCCGGTCGCCGGCGGCGTGTGGGGCCGCGCCTTC 3'

## MATERIALS AND METHODS

### Strains and growth conditions

The Bordetella pertussis strains 134 (atypical LPS, fim 3) and 509 (typical LPS, fim 2) are the two strains which are used to prepare the Dutch "whole-cell vaccine" (WCV) (P.A. van Hemert, Specific properties of acid precipitated pertussis vaccines. Progr.Immunobiol. Standard 3, (1969), pp. 297-301).

The Tohama strain was supplied by Ch. R. Manclark (FDA, Bethesda, USA).

The BP-TOX6 strain was supplied by R. Rappuoli (Sclavo Research Center) and has already been described in Relman, C.A. et al., Filamentous hemagglutinin of Bordetella pertussis: nucleotide sequence and crucial role in adherence, Proc.Natl.Acad.Sci. 86 (1989), pp. 2637-2641.

The W28 strain and W28 (69K⁻) strain were supplied by G. Doughan (Wellcome Ltd., England). In this case the 18323 strain was used as the control strain (see Relman et al., Proc.Natl.Acad.Sci. 86 (1989), p. 2637, Sato et al., Infect.Immun. 41 (1983), p. 313 and Kendrick et al., Am.J.Publ.Health 37 (1947), p. 803).

Mutants of strain 509 were obtained with the aid of a colony blotting using monoclonal antibodies. After culturing for three days on Bordet-Gengou agar plates (Difco) the colonies were blotted on nitrocellulose filters and examined for the presence of fimbriae, FHA and 92kDa OMP. The fimbriae⁻ and FHA⁻ mutants were easily detected in this way.

Small-scale growth of B.pertussis in fluid was carried out in shaking bottles containing Verwey medium: 14 g/l casamino acid, 0.2 g/l KCl, 1 g/l starch, 0.5 g/l $KH_2PO_4$, 0.1 g/l $MgCl_2.H_2O$, 0.02 g/l nicotinic acid and 0.01 g/l glutathion. 0.5 g/l nicotinic acid was added for growth in the avirulent C mode.

Large-scale fermentation in fluid was carried out in 40 l, 140 l or 300 l Bilthoven units in B2 medium with pH and $PO_2$ control (P.A. van Hemert, Specific properties of acid precipitated pertussis vaccines. Progr.Immunobiol.Standard 3 (1969), pp. 297-301).

The cells were deactivated by heating at 56°C for 10 min., centrifuged at 3000 g and resuspended in a physiological saline solution.

Opacity units were detected with the aid of a reference standard. The protein determination was carried out using the BCA protein analysis reagent (Pierce) with BSA as standard.

Intracerebral (i.c.) mouse potency test

The i.c. test was carried out using the method of Kendrick et al. (Kendrick, P.L. et al., Mouse protection test in the study of pertussis vaccine, Am.J.Publ.Hlth. 37, (1947), pp. 803 ff). Male and female NIH-bred mice (10-14 g) were used.

Each vaccine was tested in four fivefold-diluted solutions (20 mice/solution) by i.p. immunisation (0.5 ml, containing 1 mg of $AlPO_4$). WCV was tested with 1 OU, 0.2, 0.04 and 0.008 OU.

Since 1 OU contains approximately 15 $\mu$g of protein, the final calculations were made for purified antigens on a comparable protein basis.

OMV, $PT_x$ and FHA were tested in 5, 1, 0.2 and 0.04 $\mu$g doses and purified antigens in 25, 5, 1 and 0.2 $\mu$g doses on the basis of trial experiments. A WCV reference of standard potency is part of each experiment.

14-16 days after immunisation, the mice were injected intracerebrally with 10 $\mu$l of a suspension of B.pertussis 18323, containing $15 \times 10^3$ bacteria. This suspension was prepared from a lyophilised culture having a $LD_{50}$ of $< 10^3$ bacteria. This culture was cultured for 4 days on Bordet-Gengou plates and then cultured for a further 24 hours on Bordet-Gengou and suspended in 1 part by weight of casamino acid in a physiological saline solution to obtain a concentration of $15 \times 10^5$ bacteria/ml.

Twelve mice were each injected with $15 \times 10^3$ bacteria.

The groups of 12 mice in each case were likewise injected with 1500, 300 and 60 bacteria. Only those mice which died from day 4 to day 14 were taken into consideration in the calculation.

On the basis of the percentage of mice which survived, the strength of the vaccine was calculated using the "probit" analysis.

The test had a confidence range of 55%-192% of the reference preparation with a confidence level of 95%.

The strength of a vaccine is calculated (after "probit" transformation) using the equation

$$\frac{\text{potency, standard}}{\text{potency, test}} = \frac{ED_{50}, \text{ test}}{ED_{50}, \text{ standard}}$$

Preparation of outer membrane vesicles (OMV)

Outer membrane vesicles (OMV) were prepared by resuspending bacteria after growth and centrifuging in 10 mM Tris, pH 8.0, 2 mM EDTA. Approximately 10 ml of Tris were used to resuspend bacteria from one litre of 100 OU/ml culture.

The suspension was sonicated on ice (50% capacity, 100 W, 50%. duty cycle, Branson 250 sonifier). After centrifuging for 10 min at 10,000 g, the supernatant was pelleted for one hour at 50,000 g. This pellet was resuspended in 1% (w/v) sarkosyl (sigma) in 10 mM Tris.HCl volume (Filip, C., et al., Solubilization of the cytoplasmic membrane of Escherichia coli by the ionic detergent sodium lauryl sarcosinate. J.Bacteriol. 255 (1973), pp. 717-722). After centrifuging for 10 min at 10,000 g, the OMVs were pelleted for one hour at 50,000 g. This method was repeated once.

The pellet finally obtained was resuspended in 10 mM Tris/HCl, pH 8.0, one-tenth of the original Tris volume. The protein concentration was determined using the BCA protein assay reagent (Pierce Chemical Company) using BSA as standard.

The desoxycholate (DOC) treatment of OMV was carried out as follows: OMVs were resuspended in 1.5% (w/v) DOC, 50 mM glycine, 5 mM EDTA, pH 9.0, sonicated for 5 min and pelleted by centrifuging: 10 min at 10,000 g followed by one hour at 100,000 g.

8

The LPS content of OMV is approximately 25% (wt/wt LPS/protein) and after treatment with DOC is about 10%, using KDO determination.

Characteristics of OMPs

The OMPs were characterised via trypsin sensitivity and Triton®-X-100/MgCl₂ extractability as described by L. van Alphen, et al., Characteristics of major outer membrane proteins of Haemophilus influenzae. J.Bacteriol. 155 (1983), pp. 878-855.

Purification of antigens

- Pertussis toxin (PT) was purified from the culture supernatant using Affigelblue® (Pharmacia) and column chromatography as described by Sekura, R.D., et al., Pertussis toxin, affinity, purification of a new ADp-ribosyltransferase. J.Biol.Chem. 258 (1983), 14647-14651. PT without enzymatic activity (9K, 129G) was supplied by R. Rappuoli [Pizza, M.G., et al., Mutants of pertussis toxin suitable for vaccine development, Science 246 (1989), 497-500].
- FHA was purified from the culture supernatant using Affigelblue® and hydroxyapatite column chromatography (Sato et al., Separation and Purification of hemagglutinins from Bordetella pertussis, Infect.Immun. 41, (1983), pp. 313-320). Purified 69kDa OMP was supplied by R. Rappuoli (Sclavo Research Center).
- Fimbriae were isolated by means of a heat shock treatment of bacteria in the presence of urea, followed by a few centrifuging steps (Mooi, F.R., et al., Characterization of fimbriae subunits from Bordetella species, Microb.Pathog. 2 (1987) pp. 473-484).
- Endotoxin was purified by phenol extraction of bacteria (Ackers, J.L. and J.M. Dolby, The antigen of Bordetella pertussis that induces bactericidal antibody and its relationship to protection of mice, J.Gen.Microbiol. 70 (1972), 371-382).
- The 92kDa, 38kDa and 32kDa OMPs were obtained from the culture-cell pellet as follows:
  The cells were extracted with 0.5 M CaCl₂, 0.14 M NaCl, 1% Zwittergent® 3-14 (Calbiochem) pH 4.0 (10 ml per gram wet weight of cells). After resuspension, the pH was adjusted to pH 5-6. After stirring for one hour at room temperature, the suspension was centrifuged (1 h, 3000 g) and the supernatant was treated with 20% (v/v) ethanol, stirred for 30 minutes and centrifuged for 30 min at 10,000 g. The supernatant was dialysed against 50 mM Tris.HCl, 0.05% Zwittergen® 3-14, pH 8.0. After centrifuging for 30 min at 10,000 g, the supernatant was introduced into a DEAE Sepharose® CL6B (Pharmacia) column and eluted using a linear gradient of 0-0.6 M NaCl in 50 mM Tris.HCl, 0.05% Zwittergent® 3-14. Fractions enriched with either 92kDa or 32kDa were combined, concentrated with polyethylene glycol 20,000 and precipitated with 80% ethanol. The pellets were dissolved in 50 mM Tris.HCl, 0.5% Zwittergent® 3-14, pH 9.0, and passed through a Sephacryl® S300 column in 50 mM Tris, 0.05% Zwittergent® 3-14, pH 8.0. Fractions containing 38kDa were combined, concentrated and stored. The 38kDa OMP was purified from C-mode cells after OMV isolation. OMV pellets were treated with 5% Zwittergent® 3-14, 50 mM Tris, pH 7.5, and stirred for 2 hours at 37°C. After centrifuging for 1 hour at 50,000 g, the supernatant was passed through a Sephacryl® S300 column in 0.05% Zwittergent® 3-14, 50 mM Tris, pH 8.0. Fractions containing 38kDa were combined, concentrated and stored.

Preparation of monoclonal antibodies

Ba1B/c mice were immunised i.p. with 20 μg purified antigens or OMV in the presence of 0.5 mg of AlPO₄. (AlPO₄ gel was prepared from AlCl₃ and Na₃PO₄ in equal amounts, adjusting the pH to 7.0 using 20% (w/v) Na₂CO₃). The mice were injected four times over a period of one week. Following the final injection, the cells of the spleen were brought into contact with Sp 2/0 myeloma cells as described in Abdillahi, H and J.T. Poolman, Neisseria meningitidis group B serosubtyping using monoclonal antibodies in whole-cell ELISA. Microb.Pathog. 4, (1988), pp. 27-32 and the hybridoma cells were cultured. After adequate cell growth, the supernatants were tested via ELISA using purified antigens and OMV. Interesting hybridomas were cloned twice using the "limiting" dilution method.

Ascites fluid was obtained from these clones after introduction into the peritoneum of Pristane-injected mice. The collection of monoclonal antibodies against B.pertussis antigens is described in Poolman, J.T., et al., Description of a hybridoma bank towards Bordetella pertussis toxin and surface antigens. Microb.Pathog. 8, (1990), 377 - 382.

ELISA

One hundredth $\mu$l of 2 $\mu$g/ml antigen was coated in PBS (LPS in $Na_2CO_3$, pH 9.0) in the wells of round-bottomed polyvinyl chloride microtitre plates. Purified OMPs were diluted to maintain the concentration of Zwittergent® 3-14 below 0.001%. PT ELISA was preceded by a coating step using fetuin. The coating took place over a period of 16 hours at room temperature. The plates were washed twice with tap water + 0.02% Tween 80 (Merck). The reactions were carried out as described in Abdillahi, H. and J.T. Poolman, 1988 Neisseria meningitidis group B serosubtyping using monoclonal antibodies in whole-cell ELISA. Microb.Pathog. 4: 27-32, but 0.05% (w/v) Protifar (Nutricia) stain casein in order to block the non-specified bond. Rabbit anti-mouse peroxidase (own product) was used as second antibody.

The results were measured at 450 nm using Titertek Multiscar® (Flow Labs.). "Whole-cell" ELISA was carried out as described in Abdillahi, H. and J.T. Poolman, 1988 Neisseria meningitidis group B serosubtyping using monoclonal antibodies in whole-cell ELISA, Microb.Pathog. 4: 27-32, using Protifar.

SDS-PAGE/immunoblotting

Purified antigens, OMV and WCV were separated by SDS polyacrylamide gel electrophoresis and labelled as described in L. van Alphen et al., 1983, Characteristics of major outer membrane proteins of Haemophilus influenzae. J.Bacteriol. 155: 878-855.

Whole cells for SDS-PAGE were obtained by suspending 3.5 ml of bacteria with 1.0 absorption at 620 nm in 70 $\mu$g of water + 130 $\mu$l of sample buffer. Electroblotting on nitrocellulose paper after SDS-PAGE was carried out at 140 mA constant current for a period of 1 hour on a semi-dry electroblotter (Ancos, Denmark) in accordance with the manufacturer's instructions. The paper was washed for 30 min with a physiological saline solution + 10 mM Tris, pH 7.4, containing 0.5% Tween® 80 at 37°C in the same buffer. The paper was washed three times with the buffer, incubated with O.5% Protifar in buffer for 10 min, washed and incubated with rabbit anti-mouse peroxidase for 1 hour in buffer + 0.5%. Protifar. The paper was washed three times and incubated with a solution containing 24 mg of 3,3',5,5'-tetramethylbenzidine (TMB, Sigma), 80 mg of DONS, dioctyl sulphosuccinate, dissolved in 10 ml of 96% ethanol, added to 30 ml of 10 mM $Na_2HPO_4$, 5 mM citric acid, pH 5.0, to which 20 $\mu$l of 30% $H_2O_2$ have been added.

LEGEND

Fig. 1: This figure shows the i.c. mouse protection value related to the 92kDa OMP/38kDa OMP ratio in the OMV vaccine.

Fig. 2: OMP characterisation.
Path 1: Tohama OMV; virulent stage (X mode)
Path 2: Tohama OMV; avirulent stage (C mode)
Path 3: X-mode OMV after trypsin treatment
Path 4: OMV treated with Triton®-X-100/MgCl2 (supernatant)
Path 5: OMV treated with Triton®-X-100/MgCl2 (pellet)
Coomassie® staining

Fig. 3: OMP preparations, stained after blotting with the monoclonal mixture.
Path 1: OMV W28
Path 2: OMV W28 69⁻ mutant
Path 3: OMV 509
Path 4: OMV 509; C mode (avirulent stage)
Path 5: OMV 509; FHA⁻ mutant
Path 6: OMV 509; fim⁻ mutant
Path 7: OMV 509; stage IV
Path 8: Tohama OMV
Path 9: Tohama OMV; DOC treatment
Path 10: OMV BP-TOX6
Path 11: OMV BP-TOX6; DOC treatment
Path 12: OMV BP-TOX6; C mode
Path 13: OMV BP-TOX6; C mode; DOC treatment.

Fig. 4: This figure schematically shows the (generally known) plasmid pUC18 comprising a 3.8 kb insert containing the 32 kDa OMP gene.

**Claims**

**Claims for the following Contracting States : AT, BE, CH, DE, DK, FR, GB, IT, LI, LU, NL, SE**

1. Vaccine suitable for combating Bordetella pertussis comprising as active component, at least one OMP derived from B.pertussis, characterized in that said active component is at least one OMP having a molecular weight of about 92kDa or 32 kDa, being present in an OMV formulation or an artificial vesicle formulation.

2. Vaccine according to claim 1, characterized in that the active component is present in a mixed protein-detergent formulation.

3. Vaccine according to claim 2, characterized in that the detergent is Zwittergent® 3-14.

4. Vaccine according to one or more of claims 1-3, characterized in that an adjuvans is added.

5. Vaccine according to claim 4, characterized in that the adjuvans is $AlPO_4$.

6. Vaccine according to one or more of claims 1-5, characterized in that the active component comprises both OMPs having a molecular weight of about 92kDa and 32kDa.

7. Vaccine according to one or more of claims 1-6, characterized in that the active component comprises OMPs having a molecular weight of 92kDa, 32kDa and 38kDa, the 92kDa OMP/38kDa OMP weight ratio being $\geq$ 0.25.

8. Vaccine according to claim 7, characterized in that the 92kDa OMP/38kDa OMP weight ratio $\geq$ 0.4.

9. Vaccine according to one or more of claims 1-8, characterized in that the OMVs have been subjected to a treatment with an effective amount of desoxycholate in order to lower the endotoxin content in the OMVs.

10. Vaccine according to one or more of claims 1-9, characterized in that the B.pertussis strain has been chosen from the group comprising B.pertussis strain 134, B.pertussis strain 509, B.pertussis Tohama strain and B.pertussis strain BP-TOX6.

11. Vaccine according to one or more of claims 1-10, characterized in that the OMPs are derived from Bordetella pertussis strains or other micro-organisms in which the presence of 92kDa and 32kDa OMPs has been increased via genetic engineering.

12. Vaccine according to one or more of claims 1-11, characterized in that the OMVs are derived from fimbriae⁻ and/or FHA⁻ B.pertussis strains.

13. Vaccine according to claim 12, characterized in that the OMVs are derived from a fim⁻ mutant or a FHA⁻ mutant of the B. pertussis strain BP509.

14. Vaccine according to one or more of claims 1-13, characterized in that PT toxoid or FHA, or PT toxoid and FHA, are present, optionally in the form of a divalent conjugate, as additional active component(s).

15. Vaccine according to claim 14, characterized in that the PT toxoid used in the vaccine has been obtained by means of a formaldehyde treatment or glutaraldehyde treatment of PT.

16. Vaccine according to claim 14, characterized in that the PT toxoid has been derived from a B.pertussis strain in which the PT gene has been modified in such a way that it codes for an immunologically active PT having no toxic effect or a toxic effect acceptable for vaccine use.

17. Vaccine according to claim 14, characterized in that the FHA has been purified with the aid of chromatography using hydroxyapatite.

**18.** An effective amount of the vaccine according to one or more of Claims 1-17 for use in a method for the vaccination of humans, in particular children less than 2 years old.

**19.** An effective amount of the vaccine according to one or more of Claims 1-17 for use in a method according to Claim 18, in which the amount of vaccine to be injected contains a dose of 5-25 $\mu$g OMV.

**20.** OMVs derived from B.pertussis, suitable for use in the preparation of vaccines, defined in one or more of Claims 1-17.

**Claims for the following Contracting States : ES, GR**

**1.** A process for the production of a vaccine suitable for combating Bordetella pertussis comprising as active component, at least one OMP derived from B.pertussiso, characterized by bringing at least one OMP having a molecular weight of about 92kDa or 32 kDa as the active component in an OMV formulation or an artificial vesicle formulation.

**2.** The process according to claim 1, characterized in that the active component is present in a mixed protein-detergent formulation.

**3.** The process according to claim 2, characterized in that the detergent is Zwittergent$^R$ 3-14.

**4.** The process according to one or more of claims 1-3, characterized in that an adjuvans is added.

**5.** The process according to claim 4, characterized in that the adjuvans is AlPO$_4$.

**6.** The process according to one or more of claims 1-5, characterized in that the active component comprises both OMPs having a molecular weight of about 92kDa and 32kDa.

**7.** The process according to one or more of claims 1-6, characterized in that the active component comprises OMPs having a molecular weight of 92kDa, 32kDa and 38kDa, the 92kDa OMP/38kDa OMP weight ratio being $\geq$ 0.25.

**8.** The process according to claim 7, characterized in that the 92kDa OMP/38kDa OMP weight ratio $\geq$ 0.4.

**9.** The process according to one or more of claims 1-8, characterized by subjecting the OMVs to a treatment with an effective amount of desoxycholate in order to lower the endotoxin content in the OMVs.

**10.** The process according to one or more of claims 1-9, characterized in that the B.pertussis strain has been chosen from the group comprising B.pertussis strain 134, B.pertussis strain 509, B.pertussis Tohama strain and B.pertussis strain BP-TOX6.

**11.** The process according to one or more of claims 1-10, characterized in that the OMPs are derived from Bordetella pertussis strains or other micro-organisms in which the presence of 92kDa and 32kDa OMPs has been increased via genetic engineering.

**12.** The process according to one or more of claims 1-11, characterized in that the OMVs are derived from fimbriae$^-$ and/or FHA$^-$ B.pertussis strains.

**13.** The process according to claim 12, characterized in that the OMVs are derived from a fim$^-$ mutant or a FHA$^-$ mutant of the B. pertussis strain BP509.

**14.** The process according to one or more of claims 1-13, characterized by using PT toxoid or FHA, or PT toxoid and FHA, optionally in the form of a divalent conjugate, as additional active component(s).

**15.** The process according to claim 14, characterized in that the PT toxoid used in the vaccine has been obtained by means of a formaldehyde treatment or glutaraldehyde treatment of PT.

12

16. The process according to claim 14, characterized in that the PT toxoid has been derived from a B.pertussis strain in which the PT gene has been modified in such a way that it codes for an immunologically active PT having no toxic effect or a toxic effect acceptable for vaccine use.

17. The process according to claim 14, characterized in that the FHA has been purified with the aid of chromatography using hydroxyapatite.

18. A process for the production of an effective amount of the vaccine according to one or more of claims 1-17 for use in a method for the vaccination of humans, in particular children less than 2 years old.

19. A process for the production of an effective amount of the vaccine according to one or more of claims 1-17 for use in a method according to claim 18, in which the amount of vaccine to be injected contains a dose of 5-25 $\mu$g OMV.

20. A process for the preparation of OMVs derived from B.pertussis in a way known per se, suitable for use in the preparation of vaccines, defined in one or more of Claims 1-17.

**Patentansprüche**
**Patentansprüche für folgende Vertragsstaaten : AT, BE, CH, DE, DK, FR, GB, IT, LI, LU, NL, SE**

1. Impfstoff zur Bekämpfung von Bordetella pertussis, welcher als Wirkstoff zumindest ein aus B. pertussis abgeleitetes OMP enthält, dadurch gekennzeichnet, dass der genannte Wirkstoff zumindest ein OMP mit einem Molekularwicht von 92kDA oder 32 KDA ist, welcher in einer OMV-Formel oder einer Kunstbläschen-Formel vorhanden ist.

2. Impfstoff nach Anspruch 1, dadurch gekennzeichnet, dass der Wirkstoff in der Formel eines Misch-Protein-Detergens enthalten ist.

3. Impfstoff nach Anspruch 2, dadurch gekennzeichnet, dass das Detergens Zwittergent$^R$ 3-14 ist.

4. Impfstoff nach einem oder mehreren der Ansprüche 1-3, dadurch gekennzeichnet, dass ein Adjuvans zugesetzt wird.

5. Impfstoff nach Anspruch 4, dadurch gekennzeichnet, dass das Adjuvans AlPO$_4$ ist.

6. Impfstoff nach einem oder mehreren der Ansprüche 1-5, dadurch gekennzeichnet, dass der Wirksstoff beide OMPs mit einem Molekulargewicht von etwa 92kDa und 32kDa enthält.

7. Impfstoff nach einem oder mehreren der Ansprüche 1-6, dadurch gekennzeichnet, dass der Wirksstoff OMPs mit Molekulargewichten von 92kDa, 32kDa und 38kDa enthält, wobei das Gewichtsverhältnis von 92kDa OMP zu 38kDa OMP $\geq$0.25 beträgt.

8. Impfstoff nach Anspruch 7, dadurch gekennzeichnet, dass das Gewichtsverhältnis von 92kDA OMP zu 38kDa OMP $\geq$ 0.4 beträgt.

9. Impfstoff nach einem oder mehreren der Ansprüche 1-8, dadurch gekennzeichnet, dass die OMVs einer Behandlung mit einer wirksamen Menge an Desoxycholat zur Herabsetzung des Endotoxingehalts in den OMVs unterzogen werden.

10. Impfstoff nach einem oder mehreren der Ansprüche 1-9, dadurch gekennzeichnet, dass der B. pertussis-Stamm aus der Gruppe ausgewählt wurde, welcher den B.pertussis Stamm 134, den B. pertussis-Stamm 509, den B. pertusissis-Tohama-Stamm und den B. pertussis-Stamm BP-TOX6 enthält.

11. Impfstoff nach einem oder mehreren der Ansprüche 1-10, dadurch gekennzeichnet, dass die OMPs aus den Bordetella pertussis-Stämmen oder anderen Mikroorganismen abgeleitet werden, in denen der Gehalt an 92kDa und 32kDa-OMPs über genetische Steuerung erhöht wird.

**12.** Impfstoff nach einem oder mehreren der Ansprüche 1-11, dadurch gekennzeichnet, dass die OMWs aus Fimbrien⁻ und/oder FHA⁻ B. pertussis-Stämmen abgeleitet werden.

**13.** Impfstoff nach Anspruch 12, dadurch gekennzeichnet, dass die OMWs aus einem Fim⁻ oder einem FHA⁻Mutanten des B. pertussis-Stamms BP509 abgeleitet werden.

**14.** Impfstoff nach einem oder mehreren der Ansprüche 1-13, dadurch gekennzeichnet, dass das PT-Toxoid oder FHA bzw. das PT-Toxoid und FHA wahlweise in Form eines zweiwertigen Konjugats als zusätzliche Wirkstoffe vorhanden sind.

**15.** Impfstoff nach Anspruch 14, dadurch gekennzeichnet, dass das im Impfstoff verwendete PT-Toxoid mit Hilfe einer Formaldehyd- oder Glutaraldehydbehandlung des PT erzielt wird.

**16.** Impfstoff nach Anspruch 14, dadurch gekennzeichnet, dass das PT-Toxoid aus einem B.pertussis-Stamm abgeleitet wird, in welchem das PT-Gen so verändert wird, dass sich ein immunologisch wirksames PT ergibt, welches keinerlei toxische Wirkung oder aber eine zur Verwendung als Impfstoff tolerierbare toxische Wirkung aufweist.

**17.** Impfstoff nach Anspruch 14, dadurch gekennzeichnet, dass das FHA mit Hilfe von Chromatographie unter Verwendung von Hydroxyapatit gereinigt wird.

**18.** Wirksame Menge an Impfstoff nach einem oder mehreren der Ansprüche 1-17 zur Verwendung in einem Verfahren zur Impfung von Personen, insbesondere von Kindern im Alter von unter 2 Jahren.

**19.** Wirksame Menge an Impfstoff gemäss einem oder mehreren der Ansprüche 1-17 zur Verwendung in einem Verfahren nach Anspruch 18, bei welchem die zu injizierende Impfstoffmenge eine Dosis von 5-15 $\mu$g OMV enthält.

**20.** Aus B. pertussis abgeleitete OMVs, welche sich zur Herstellung von Impfstoffen gemäss Definition nach einem oder mehreren der Ansprüche 1-17 eignen.

**Patentansprüche für folgende Vertragsstaaten : ES, GR**

**1.** Verfahren zur Herstellung eines Impfstoffs zur Bekämpfung von Bordetella pertussis, welcher als Wirkstoff zumindest ein aus B. pertussis abgeleitetes OMP enthält, dadurch gekannzeichnet, dass zumindest ein OMP mit einem Molekulargewicht von etwa 92kDa oder 32 kDA als Wirkstoff in eine OMV-Formel oder eine Kunstbläschen-Formel eingebracht wird.

**2.** Verfahren nach Anspruch 1, dadurch gekennzeichnet, dass der Wirkstoff in der Formel eines Misch-Protein-Detergens enthalten ist.

**3.** Verfahren nach Anspruch 2, dadurch gekennzeichnet, dass das Detergens Zwittergent$^R$ 3-14 ist.

**4.** Verfahren nach einem oder mehreren dar Ansprüche 1-3, dadurch gekennzeichnet, dass ein Adjuvans zugesetzt wird.

**5.** Verfahren nach Anspruch 4, dadurch gekennzeichnet, dass das Adjuvans $AlPO_4$ ist.

**6.** Verfahren nach einem oder mehreren der Ansprüche 1-5, dadurch gekennzeichnet, dass der Wirkstoff beide OMPs mit einem Molekulargewicht von etwa 92kDa und 32kDa enthält.

**7.** Verfahren nach einem oder mehreren der Ansprüche 1-6, dadurch gekennzeichnet, dass der Wirkstoff OMPs mit Molekulargewichten von 92kDa, 32kDA und 38kDa enthält, wobei das Gewichtsverhältnis von 92kDa OMP zu 38Da OMP $\geq$0.25 beträgt.

**8.** Verfahren noch Anspruch 7, dadurch gekennzeichnet, dass das Gewichtsverhältnis von 92kDa OMP zu 38kDA OMP $\geq$0.4 beträgt.

**9.** Verfahren noch einem oder mehreren der Ansprüche 1-8, dadurch gekennzeichnet, dass die OMVs einer Behandlung mit einer wirksamen Menge an Desoxycholat zur Herabsetzung des Endotoxingehaltes in den OMVs unterzogen werden.

**10.** Verfahren nach einem oder mehreren der Ansprüche 1-9, dadurch gekennzeichnet, dass der B. pertussis-Stamm aus der Gruppe ausgewählt wurde, welche den B. pertussis-Stamm 134, den B. pertussis-Stamm 509, den B. pertussis-Tohama-Stamm und den B. pertussis-Stamm BP-TOX6 enthält.

**11.** Verfahren nach einem oder mehreren der Ansprüche 1-10, dadurch gekennzeichnet, dass die OMPs aus den Bordetella pertussis-Stämmen oder anderen Mikroorganismen abgeleitet werden, in denen der Gehalt an 92kDa und 32kDa-OMPs über genetische Steuerung erhöht wurde.

**12.** Verfahren nach einem oder mehreren der Ansprüche 1-11, dadurch gekennzeichnet, dass die OMVs aus Fimbrien⁻ und/oder FHA⁻ B. pertussis-Stämmen abgeleitet werden.

**13.** Verfahren nach Anspruch 12, dadurch gekennzeichnet, dass die OMWs aus einem Fim⁻ oder einem FHA⁻ Mutanten des B. pertussis-Stammes BP509 abgeleitet werden.

**14.** Verfahren nach einem oder mehreren der Ansprüche 1-13, dadurch gekennzeichnet, dass PT-Toxoid oder FHA bzw. PT-Toxoid und FHA wahlweise in Form eines zweiwertigen Konjugate als zusätzliche wirkstoffe verwendet werden.

**15.** Verfahren nach Anspruch 14, dadurch gekennzeichnet, dass das in Impfstoff verwendete PT-Toxoid mit Hilfe einer Formaldehyd- oder Glutaraldehydbehandlung des PT erhalten wird.

**16.** Verfahren nach Anspruch 14, dadurch gekennzeichnet, dass das PT-Toxoid aus einem B. pertussis-Stamm abgeleitet wird, in welchem das PT-Gen so verändert wird, dass sich ein immunologisch wirksamen PT ergibt, welches keinerlei toxische Wirkung oder aber eine zur Verwendung als Impfstoff tolerierbare toxische Wirkung aufweist.

**17.** Verfahren nach Anspruch 14, dadurch gekennzeichnet, dass das FHA mit Hilfe von Chromatographie unter Verwendung von Hydroxyapatit gereinigt wird.

**18.** Verfahren zur Herstellung einer wirksamen Menge an Impfstoff noch einem oder mehreren der Ansprüche 1-17 zur Verwendung in einem Verfahren zur Impfung von Personen, insbesondere von Kindern im Alter von unter 2 Jahren.

**19.** Verfahren zur Herstellung einer wirksamen Menge an Impfstoff nach einem oder mehreren der Ansprüche 1-17 zur Verwendung in einem Verfahren nach Anspruch 18, bei welchem die zu injizierende Impfstoffmenge eine Dosis von 5-25 $\mu$g OMV enthält.

**20.** Verfahren zur Herstellung von aus B. pertussis in an sich bekannter Weise abgeleiteten OMVs, welche sich zur Herstellung von Impfstoffen gemäss Definition nach einem der Ansprüche 1-17 eignen.

**Revendications**

**Revendications pour les Etats contractants suivants : AT, BE, CH, DE, DK, FR, GB, IT, LI, LU, NL, SE**

**1.** Vaccin adapté pour la lutte contre Bordetella pertussis comprenant en tant que constituant actif, au moins un OMP dérivée de B. pertussis, caractérisé en ce que ledit constituant actif est au moins une OMP ayant une masse moléculaire d'environ 92 kDa ou 32 kDa, et étant présent dans une formulation de type OMV ou une formulation de vésicule artificielle.

**2.** Vaccin selon la revendication 1, caractérisé en ce que le constituant actif est présent dans une formulation mixte de type protéine-détergent.

**3.** Vaccin selon la revendication 2, caractérisé en ce que le détergent est le Zwittergent® 3-14.

**4.** Vaccin selon une ou plusieurs des revendications 1 à 3, caractérisé en ce qu'un adjuvant est ajouté.

5. Vaccin selon la revendication 4, caractérisé en ce que l'adjuvant est AlPO$_4$.

6. Vaccin selon une ou plusieurs des revendications 1 à 5, caractérisé en ce que le constituant actif comprend les deux OMP ayant une masse moléculaire d'environ 92 kDa et 32 kDa.

7. Vaccin selon une ou plusieurs des revendications 1 à 6, caractérisé en ce que le constituant actif comprend des OMP ayant une masse moléculaire de 92 kDa, 32 kDa et 38 kDa, le rapport pondéral OMP de 92 kDa/OMP de 38 kDa étant supérieur ou égal à 0,25.

8. Vaccin selon la revendication 7, caractérisé en ce que le rapport pondéral OMP de 92 kDa/OMP de 38 kDa est supérieur ou égal à 0,4.

9. Vaccin selon une ou plusieurs des revendications 1 à 8, caractérisé en ce que les OMV ont été soumises à un traitement avec une quantité efficace de desoxycholate afin d'abaisser la teneur en endotoxine des OMV.

10. Vaccin selon une ou plusieurs des revendications 1 à 9, caractérisé en ce que la souche de B. pertussis a été choisie parmi le groupe comprenant le souche de B.pertussis 134, la souche de B. pertussis 509, la souche de B. pertussis Tohams et la souche de B. pertussis BP-TOX6.

11. Vaccin selon une ou plusieurs des revendications 1 à 10, caractérisé en ce que les OMP sont dérivées des souches de Bordetella pertussis ou d'autres micro-organismes dans lesquels la présence des OMP de 92 kDa et de 32 kDa a été accrue par génie génétique.

12. Vaccin selon une ou plusieurs des revendications 1 à 11, caractérisé en se que les OMV sont dérivées des souches de B. pertussis fimbriae$^-$ et/ou FHA$^-$ .

13. Vaccin selon la revendication 12, caractérisé en ce que les OMV sont dérivées d'un mutant fim$^-$ ou d'un mutant FHA$^-$ de la souche de B.pertussis BP509.

14. Vaccin selon une ou plusieurs des revendications 1 à 13, caractérisé en ce qu'une toxoïde PT ou FHA, ou une toxoïde PT et FHA, sont présents, éventuellement sous forme d'un conjugué divalent, en tant que constituant (s) actif (s) supplémentaire (s).

15. Vaccin selon la revendication 14, caractérisé en ce que la toxoïde PT utilisée dans le vaccin a été obtenue par traitement au formaldéhyde ou au glutaraldéhyde de PT.

16. Vaccin selon la revendication 14, caractérisé en ce que la toxoïde PT a été obtenue à partir d'une souch de B. pertussis dans laquelle le gène PT a été modifié de telle manière qu'il code pour une PT immunologiquement active n'ayant aucun effet toxique ou ayant un effet toxique acceptable pour l'utilisation dans un vaccin.

17. Vaccin selon la revendication 14, caractérisé en ce que la FHA a été purifié à l'aide d'une chromatographie utilisant de l'hydroxyapatite.

18. Quantité efficace du vaccin selon une ou plusieurs des revendications 1 à 17 à utiliser dans un procédé de vaccination des humains, en particulier des enfant âgés de moins de deux ans.

19. Quantité efficace du vaccin selon une ou plusieurs des revendications 1 à 17 à utiliser dans un procédé selon la revendication 18, dans lequel la quantité de vaccin à injecter contient une dose d'OMV de 5 à 25 $\mu$g.

20. OMV dérivées de B.pertussis, pouvant être convenablement utilisées dans la préparation des vaccins définis dans une ou plusieurs des revendications 1 à 17.

**Revendications pour les Etats contractants suivants : ES, GR**

1. Procédé de prépartion d'un vaccin adapté pour la lutte contre Bordetella pertussis comprenant an tant que constituant actif, au moins une OMP dérivée de B. pertussis, caractérisé en ce que l'on apporte comme constituant actif au moins une OMP ayant une masse moléculaire d'environ 92 kDa ou 32 kDa, dans un formulation de type OMV ou une formulation de vésicule artificielle.

2. Procédé selon la revendication 1, caractérisé en ce que le constituant actif est présent dans une fomulation mixte de type protéine-détergent.

3. Procédé selon la revendication 2, caractérisé en ce que le détergent est le Zwittergent ® 3-14.

4. Procédé selon une ou plusieurs des revendications 1 à 3, caractérisé en ce qu'un adjuvant est ajouté.

5. Procédé selon la revendication 4, caractérisé en ce que l'adjuvant est $AlPO_4$.

6. Procédé selon une ou plusieur des revendications 1 à 5, caractérisé en ce que le constituant actif comprend le deux OMP ayant une masse moléculaire d'environ 92 kDa et 32 kDa.

7. Procédé selon une ou plusieurs des revendications 1 à 6, caractérisé en ce que le constituant actif comprend des OMP ayant une masse moléculaire de 92 kDa, 32 kDa et 38 kDa, le rapport pondéral OMP de 92 kDa/OMP de 38 kDa étant supérieur ou égal à 0,25.

8. Procédé selon la revendication 7, caractérisé en ce que le rapport pondéral OMP de 92 kDa/OMP de 38 kDa est supérieur ou égal à 0,4.

9. Procédé selon une ou plusieurs des revendications 1 à 8, caractérisé en ce que le OMV ont été soumises à un traitement avec une quantité efficace de desoxycholate afin d'abaisser la teneur en endotoxine des OMV.

10. Procédé selon une ou plusieurs des revendications 1 à 9, caractérisé en ce que la souche de B. pertussis a été choisie parmi le groupe comprenant la souche de B. pertussis 134, la souche de B. pertussis 509, la souche de B. pertussis Tohams et la souche de B. pertussis BP-TOX6.

11. Procédé selon une ou plusieurs des revendications 1 à 10, caractérisé en ce que les OMP sont dérivées des souches de Bordetella pertussis ou d'autres micro-organismes dans lesquels la présence des OMP de 92 kDa et de 32 kDa a été accrue par génie génétique.

12. Procédé selon une ou plusieurs des revendications 1 à 11, caractérisé en ce que les OMV sont dérivées des souches de B. pertussis fimbriae⁻ et/ou FHA⁻.

13. Procédé selon la revendication 12, caractérisé en ce que les OMV sont dérivées d'un mutant fim⁻ ou d'un mutant FHA⁻ de la souche de B. pertussis BP509.

14. Procédé selon une ou plusieurs des revendications 1 à 13, caractérisé en ce qu'une toxoïde PT ou FHA, ou une toxoïde PT et FHA, sont présents éventuellement sous forme d'un conjugué divalent, en tant que constituant (s) actif (s) supplémentaire(s).

15. Procédé selon la revendication 14, caractérisé en ce que la toxoïde PT utilisée dans le vaccin a été obtenue par traitement au formaldéhyde ou au glutaraldéhyde de PT.

16. Procédé selon la revendication 14, caractérisé en ce que la toxoïde PT a été obtenue à partir d'une souche de B. pertussis dans laquelle le gène PT a été modifié de telle manière qu'il code pour une PT immunologiquement active n'ayant aucun effet toxique ou ayant un effet toxique acceptable pour l'utilisation dans un vaccin.

17. Procédé selon la revendication 14, caractérisé en ce que la FHA a été purifié à l'aide d'une chromatographie utilisant de l'hydroxyapatite.

**18.** Procédé de préparation d'une quantité efficace du vaccin selon une ou plusieurs des revendications 1 a 17 à utiliser dans un procédé de vaccination des humains, en particulier des enfants âgés de moins de deux ans,

**19.** Procédé de préparation d'une quantité efficace du vaccin selon une ou plusieurs des revendications 1 à 17 a utiliser dans un procède selon la revendication 18, dans lequel la quantite de vaccin à injecter contient une dose d'OMV de 5 à 25 μg.

**20.** Procédé de préparation d'OMV dérivées de B. pertussis, d'une façon connue en soi, pouvant être convenablement utilisées dans la préparation des vaccins définis dans une ou plusieurs des revendications 1 à 17.

# Fig-1    I.C.POTENCY <--> 92k/38K RATIO

+    92K/38 ratio

fig-2

fig - 3

# fig-4